# EUROPEAN PATENT SPECIFICATION

(11) **EP 3 226 818 B1**
(45) Date of publication and mention of the grant of the patent: **01.01.2020**
(21) Application number: 15841076.1
(22) Date of filing: 30.11.2015
(51) Int. Cl.: A61F 5/443, A61F 5/445

(54) **HOLDER FOR A STOMA DRESSING**
HALTER FÜR EINEN STOMAVERBAND
SUPPORT POUR UN PANSEMENT DE STOMIE

(30) Priority: 02.12.2014 NL 2013903
(43) Date of publication of application: 11.10.2017
(73) Proprietor: Stomydo B.V., 5953 KL Reuver (NL)
(72) Inventor: COX, Peter Jacobus Marie, 5953 KL Reuver (NL)
(74) Representative: Algemeen Octrooi- en Merkenbureau B.V.
(86) International application number: PCT/NL2015/050835
(87) International publication number: WO 2016/089205

(56) References cited:
- WO-A1-95/11644
- WO-A1-2014/036424
- WO-A2-01/03640
- KR-Y1- 0 120 946
- US-A- 4 686 355
- US-A- 6 018 143
- US-A1- 2013 245 584
- US-B1- 7 316 313

## Description

The invention relates to a holder for a stoma dressing in combination with a stoma dressing.

A stoma is an artificial opening in the human or animal body and can function to discharge faecal matter or urine or bile or to facilitate the respiration. In the latter case the term "tracheostoma" is used. Where in the present application the term "stoma" is used, however, a stoma is meant which is placed to facilitate the discharge of faecal matter and urine. In such situations, the term "ileostoma" is used to indicate a stoma that is connected to the small intestine, or "colostoma", to indicate a stoma that is connected to the large intestine, or "urostoma", to indicate a stoma that functions to discharge urine.

Usually the stoma wound is covered by a stoma dressing (also called collecting material or skin flange), possibly provided with an ostomy pouch for collecting the faecal matter. It will be understood that said dressing (or skin flange) must be replaced at regular intervals, on which occasion the stoma wound must be cleaned.

Such a stoma dressing is known, for example from International patent publication WO2013/142577. Such a stoma dressing usually comprises two wall surfaces secured together along their circumferential edges to define a collection cavity. One of the wall surfaces is provided with an opening to be placed around the stoma, such that faecal matter can be received in the collection cavity. To that end an adhesive barrier is provided around the opening in the wall surface, so that an adequate, durable and above all moisture-tight connection of the stoma dressing to the skin directly surrounding the stoma wound is ensured.

In another type of stoma dressing, a flange is applied around the stoma wound, to which flange a separate ostomy pouch can be connected. The ostomy pouch is in that case replaced more often, whilst the flange is only exchanged after 2-3 days.

WO2004/036424 discloses a container which can be placed on the body of a stoma patient in which container an ostomy bandage can be received, while it is coupled around the stoma wound.

WO01/03640 discloses a belt provided with a holder in which a medical device can be received.

US2013/0245584 discloses a belt provided with a container in which bottles can be received, which bottles are intended for collecting wound fluid.

Generally, however, stoma dressings are intended for single or short use and must be exchanged regularly, therefore. When the stoma dressing is being exchanged, the clean stoma dressing adheres best to the body (to the skin surrounding the stoma wound) if the temperature of the stoma dressing, and in particular of the adhesive barrier, is about the same as the skin temperature.

The stoma patient is to that end advised to heat the clean stoma dressing by rubbing it with his hands or temporarily putting the dressing under his armpits or under his waistband.

WO2014/036424 A1 discloses a holder for a stoma dressing.

International patent publication no. WO95/011644 A1 discloses a style having a heated tip for use as an auxiliary device in this regard. The user must touch the location of the adhesive barrier of the already applied clean stoma dressing with the heated style so as to heat the adhesive barrier and thus realise the desired adhesive effect.

The application method proposed therein is laborious, however, and uncomfortable for the stoma patient, since displacement or incomplete local heating of the adhesive barrier will lead to an incorrect, not very durable and above all not moisture-tight connection of the stoma dressing to the skin surrounding the stoma wound.

The object of the invention is to provide a solution to the above-described drawbacks, and in order to achieve that object a holder for a stoma dressing in combination with a stoma dressing as described in claim 1 is proposed which comprises an envelope which encloses a space, in which space the stoma dressing can be placed, as well as heating means for heating the stoma dressing.

In this way a uniform and complete heating of the stoma dressing prior to the application thereof around the stoma wound is realised, so that the stoma dressing can be correctly applied around the stoma wound in one go without displacement of the dressing, whilst furthermore an adequate, durable and above all moisture-tight connection is obtained. Preferred embodiments of the invention are defined in the dependent claims.

The envelope may in particular be closable, in which case the closable envelope is provided with a zip fastener or one or more press-studs, or with a Velcro closure or a magnetic closure. In addition to the fact that in this way a quick(er) and (more) uniform heating of the stoma dressing to the desired treatment temperature (skin temperature) is realised, the use of a closable envelope for storing and pre-heating stoma dressings is also desirable for hygienic reasons. Being able to close the holder is furthermore desirable for reasons of thermal insulation.

With a view to realising a quick and effective heat transfer to the stoma dressing, the heating means are provided in the envelope at the location of this space. In this way there is substantially direct contact between the heating means and the stoma dressing present in the envelope, which reduces the heating time.

The heating means comprise one or more heating wires worked into the envelope. In this way a direct and effective heat transfer is ensured, and in addition the entire space in which the stoma dressing is present is heated, thus guaranteeing a uniform heating of the stoma dressing.

The latter leads to an adequate, durable and above all moisture-tight connection of the stoma dressing to the skin surrounding the stoma wound.

The heating means comprise a power supply, which is connected to the heating wires. The power supply is capable of being switched on and off; preferably it is also rechargeable.

In another embodiment, the envelope is provided with a further envelope, in which the power supply can be accommodated.

In addition to that, in another embodiment the heating means are provided with heating time setting means for setting a heating time, or with temperature setting means for setting a heating temperature. In this way the holder can be adjusted by the stoma patient in a user-friendly manner, making it possible to set the heating time on the one hand or a maximum heating temperature on the other hand. In another functional embodiment, the duty cycle can be set as a percentage, for example 100%-75%-50%-25% of the output of the power supply, thereby realising a pulsed heating action by the heating means.

In all cases the stoma dressing is prevented from being heated too long or to a too high temperature, which might cause damage to the skin upon application of the stoma dressing.

In another functional embodiment, the envelope is made of a fabric, so that it is easy to produce.

The invention will now be explained in more detail with reference to a drawing in which:
Figure 1 is a first view of an embodiment of a stoma dressing holder according to the disclosure;
Figure 2 is a second view of an embodiment of a stoma dressing holder;
Figure 3 is a detailed view of an embodiment of a stoma dressing holder;
Figure 4 is another detailed view of an embodiment of a stoma dressing holder.

For a better understanding of the invention, corresponding parts shown in the various figures will be referred to by identical numerals in the description of the figures below.

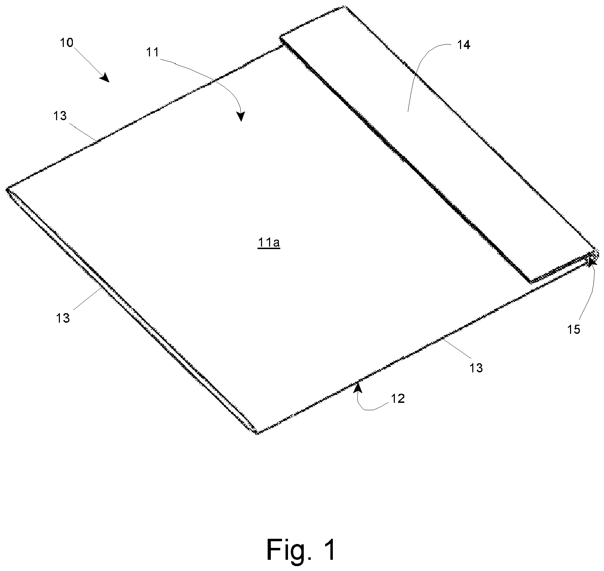

In Figure 1 a first view of an embodiment of a stoma dressing holder is shown.

The holder for the stoma dressing is indicated at 10 and forms an envelope which envelopes or encloses a space 15. The envelope is preferably made up of a first layer of fabric 11 and a second layer of fabric 12, which are joined at their circumferential edges 13. The two layers of fabric 11 and 12 are not joined at one circumferential edge portion, so that the space 15 formed by the two layers of fabric 11 and 12 can be accessed. One of the layers of fabric 11 or 12, in this case layer 12, may be provided with a flap 14 that can be folded over the open circumferential edge portion, so that the space 15 can (more or less) be closed.

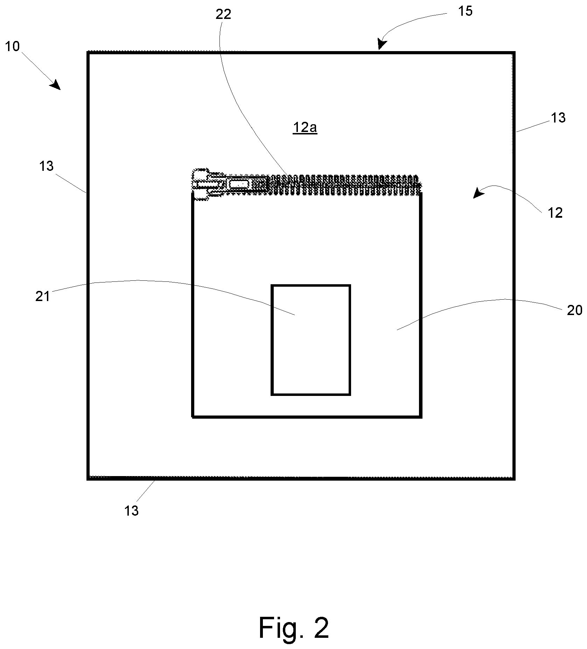

In Figure 2 another side of the stoma dressing holder is shown. The layer of fabric 12 is provided with a further layer of fabric 20 on this side, which layer of fabric 20 is provided on top of the layer of fabric 12, thus forming a further envelope. Said further envelope can preferably be closed using suitable closure means 22, in the form of a zip fastener in Figure 2. It is also possible, however, to use other closure means 22, for example a press-stud or a magnetic closure, or a Velcro-based closure. Said further additional layer of fabric 20 is provided with a see-through window 21, whose function will be explained hereinafter.

Although the holder 10 is described herein as an envelope made up of two first and second layers of fabric 11 and 12, respectively, which are joined near their circumferential edges 13, the layers 11 and 12 need not be made of a fabric. The two layers 11 and 12 may also be made of a different kind of material, for example plastic. Furthermore, the layers 11 and 12 may be made of one double-folded strip (of fabric), with one of the joined circumferential edges 13 forming the folded edge.

The envelope described with reference to Figures 1 and 2 is intended to receive a stoma dressing. Stoma dressings are provided with an opening that is to be placed around the stoma wound, and with an adhesive barrier for attachment to the skin directly surrounding the stoma wound. Such stoma dressings are usually intended for single or short use and must be exchanged regularly, therefore. It has been found that when the stoma dressing is exchanged, the clean stoma dressing adheres best to the body if the temperature of the stoma dressing, and in particular of the adhesive barrier around the stoma dressing of the collection cavity, is about the same as the temperature of the patient's skin.

The holder 10 is for that purpose provided with heating means for heating the clean stoma dressing, which is to that end temporarily placed in the space 15 of the envelope holder 10.

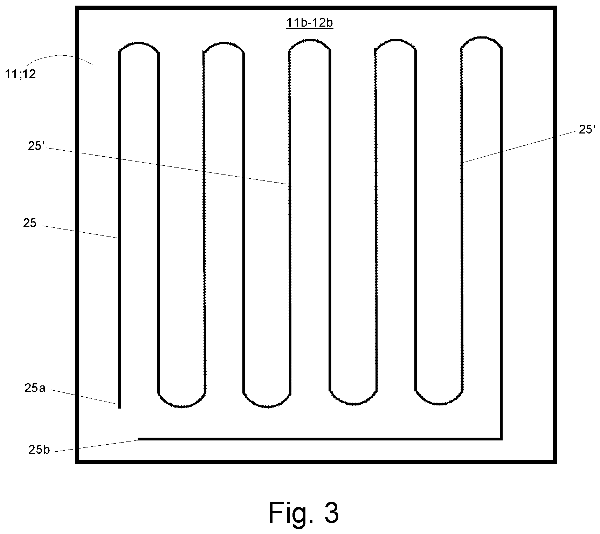

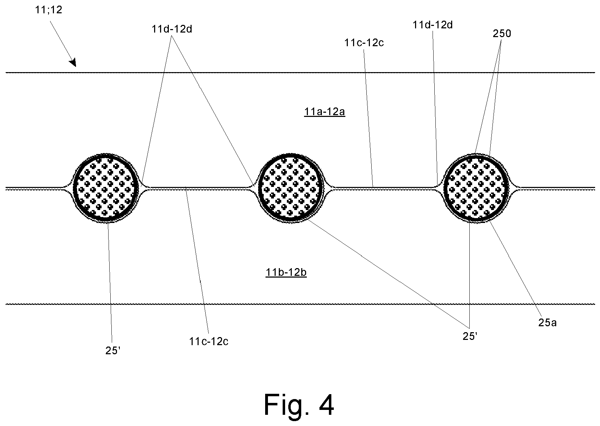

An embodiment of the heating means is shown in Figure 3 and Figure 4. The heating means shown therein comprise an electrical heating element 25, which is provided in one (or both) of the layers 11-12 of the envelope holder 10 in the form of an electrical heating wire.

For the sake of clarity of the invention, numerals 11a-12a indicate the outer side of the layers 11 and 12, respectively, whilst numerals 11b and 12b represent the inner side of the two layers, which inner sides 11b-12b face one another in the space 15. According to the invention, the heating means are provided in the inner layer 11b or 12b of the layers 11 or 12.

The electrical heating wire 25 is arranged in the form of several envelopes or windings 25' over the entire inner layer area 11b-12b of the layers 11 and/or 12. As a result, a maximum area of contact between the inner layers 11b and/or 12b and the stoma dressing present in the space 15 is realised, so that an optimum heat transfer from the electrical heating wire 25 to the stoma dressing takes place. This results in a uniform but above all quick heating of the stoma dressing, which can thus be evenly but also quickly heated to about the temperature of the patient's skin. In this way also the adhesive barrier will quickly and homogeneously reach the temperature that corresponds to the temperature of the patient's skin, resulting in a better adhesion of the stoma dressing to the skin surrounding the stoma wound and thus to an adequate, durable and above all moisture-tight connection.

It should be noted that the space 15 formed by the envelope holder 10 can be closed, preferably by means of a flap 14, as shown in Figure 1. The holder 10 may or may not be provided with additional closure means, analogously to the closure means 22 that close the further envelope 20. The closure means by which the space 15 of the envelope holder 10 can be closed may consist of a zip fastener or one or more press studs, or of a magnetic closure or, for example, a Velcro closure, in which case Velcro material will be present both on the inner side of the flap 14 and on the portion of the outer side 11a (or 12a) of the layer 11 (or 12) that is brought into contact with the flap 14.

As shown in Figure 3, the electrical heating wire 25 is worked into the envelope, and in particular into the inner side 11b-12b of the layer 11 and/or 12, in the form of loops 25'. The electrical heating wire 25 is further provided with terminal ends 25a-25b, to which a power supply (power source) can be connected. The power supply may in particular be a power supply that is capable of being switched on and off; more in particular it may be a rechargeable power supply. In a very functional embodiment, the power supply in question is accommodated in the further envelope 20, in which envelope 20 it is connected to the wire ends 25a and 25b, which to that end project from the layer of fabric 11b or 12b and extend into the further envelope 20 for being suitably connected to the terminals of the power supply. The heating wire 25 may on the other hand be a wire which is configured as a bundle comprising a large number of conducting wires.

On the other hand, the holder can be connected to a 110V/220V power source.

The power supply accommodated in the further envelope 20 may be provided with suitable setting means, such as an on-off button that can be operated by being pressed by the user. For this purpose the transparent part 21 of the further envelope 20 is used, behind which the power supply provided with the setting means can be placed, enabling the user (the stoma patient) to suitably switch the power supply on and off through the transparent window.

In the case of further functionality, the setting means are provided with time setting means, so that the power supply is activated for a certain, preferably preset heating time upon being switched on and is automatically switched off after said heating time has elapsed.

In another, suitable embodiment the heating time is preset in the setting means, which time setting is of a permanent nature. In another, more functional embodiment, the heating time can be set as desired in dependence on the type of stoma dressing and the varying properties of the associated adhesive barrier. It should be emphasised in this regard, however, that with all the different types of stoma dressings the heating times can be set so that the stoma dressing in question will have been brought to the correct temperature corresponding to the patient's skin temperature once the respective, separately adjusted heating time has lapsed.

Furthermore, the temperature can be increased in steps during a certain heating time (a fixed heating time or a heating time to be set by the user), for example by adjusting the output of the power supply in steps of 25%-50%-75%-100% of its maximum output.

In another, more complex embodiment, the setting means are provided with a temperature sensor which measures the heating temperature that prevails in the envelope 15 and automatically switches off the power supply when the desired, maximum heating temperature is reached.

In all these functional embodiments, the stoma dressing is prevented from being heated too long or to a too high temperature, which may lead to irritation or injury upon application of the stoma dressing to the skin. Nor can the stoma dressing be heated for too short a period of time, as a consequence of which the adhesive barrier would be heated insufficiently for realising an adequate, durable and above all moisture-tight connection to the skin.

Figure 4 shows in more detail an embodiment of the way in which the electrical heating wire 25 is worked into a layer 11 or 12 of an envelope holder according to the invention.

In this embodiment, each layer 11 or 12 is made up of an outer side layer 11a (12a) and an inner side layer 11b (12b), respectively. The inner side layers 11b (12b) form the space 15 in which the stoma dressing is accommodated. Between the outer side layer 11a (12a) and the inner side layer 11b (12b), the heating wire 25 is provided in several loops or windings 25', whilst the two layers 11a-11b (12a-12b) are joined at the locations indicated by numerals 11c-12c. In this way channel-shaped spaces 11d-12d are formed, in which the electrical heating wire 25 is accommodated. In this embodiment the electrical heating wire 25 is made up of a bundle comprising a large number of conducting wires 250, which may optionally be enveloped in a sheath 25a. Said large number of conductor wires 250 may also be provided in the layers 11 (12) in the form of a bundle (without a sheath 25a).

It will be understood, however, that also other types of heating wires may be worked into the layers of material 11 or 12.

## Claims

1. A combination of a holder (10) for a stoma dressing and a stoma dressing, said holder comprising an envelope composed of two layers (11, 12), which layers are joined at their circumferential edges and not joined at one circumferential edge, which envelope encloses a space (15), in which space (15) the stoma dressing is placed, **characterized in that** the holder also comprises heating means for heating the stoma dressing, wherein the heating means (25) comprise one or more heating wires (25-25'-250), which are worked into the envelope over the entire inner layer area of the envelope in the form of several envelopes or windings, thereby realising a maximum area of contact between the inner layer of the envelope and the stoma dressing being placed in the space (15).

2. The combination according to claim 1, wherein the envelope is closable.

3. The combination according to claim 2, wherein the closable envelope is provided with a zip fastener (22).

4. The combination according to claim 2, wherein the closable envelope is provided with one or more press studs.

5. The combination according to claim 2, wherein the closable envelope is provided with a magnetic closure.

6. The combination according to claim 2, wherein the closable envelope is provided with a Velcro closure.

7. The combination according to one or more of the preceding claims, wherein the heating means further comprise a power supply, which is connected to the heating wires.

8. The combination according to claim 7, wherein the power supply is capable of being switched on and off.

9. The combination according to claim 7 or 8, wherein the power supply is a 110V/220V connection.

10. The combination according to claim 7 or 8, wherein the power supply is rechargeable.

11. The combination according to claim 7, 9 or 10, wherein the envelope is provided with a further envelope (20), in which the power supply can be accommodated.

12. The combination according to one or more of the preceding claims, wherein the heating means are provided with time setting means for setting a heating time

13. The combination according to one or more of the preceding claims, wherein the heating means are provided with temperature setting means for setting a heating temperature.

14. The combination according to one or more of the preceding claims, wherein the envelope is made of a fabric.

## Patentansprüche

1. Kombination aus einem Halter (10) für einen Stoma-Verband und einem Stoma-Verband, wobei der Halter eine Hülle umfasst, die aus zwei Schichten (11, 12) zusammengesetzt ist, wobei die Schichten an ihren Umfangskanten verbunden und nicht an einer Umfangskante verbunden sind, wobei die Hülle einen Raum (15) umschließt, in welchem Raum (15) der Stoma-Verband platziert ist, **dadurch gekennzeichnet, dass** der Halter auch Heizmittel zum Erwärmen des Stoma-Verbandes umfasst, wobei die Heizmittel (25) einen oder mehrere Heizdrähte (25-25'-250) umfassen, die über den gesamten inneren Schichtbereich der Hülle in Form mehrerer Hüllen oder Wicklungen in die Hülle eingearbeitet sind, wodurch eine maximale Kontaktfläche zwischen der inneren Schicht der Hülle und dem in dem Raum (15) platzierten Stoma-Verband realisiert wird.

2. Kombination nach Anspruch 1, wobei die Hülle verschließbar ist.

3. Kombination nach Anspruch 2, wobei der verschließbare Umschlag mit einem Reißverschluss (22) versehen ist.

4. Kombination nach Anspruch 2, wobei der verschließbare Umschlag mit einem oder mehreren Druckknöpfen versehen ist.

5. Kombination nach Anspruch 2, wobei die verschließbare Hülle mit einem Magnetverschluss versehen ist.

6. Kombination nach Anspruch 2, wobei die verschließbare Hülle mit einem Klettverschluss versehen ist.

7. Kombination nach einem oder mehreren der vorstehenden Ansprüche, wobei die Heizmittel ferner eine Stromversorgung umfassen, die mit den Heizdrähten verbunden ist.

8. Kombination nach Anspruch 7, wobei die Stromversorgung ein- und ausschaltbar ist.

9. Kombination nach Anspruch 7 oder 8, wobei die Stromversorgung eine 110V/220V-Verbindung ist.

10. Kombination nach Anspruch 7 oder 8, wobei die Stromversorgung wiederaufladbar ist.

11. Kombination nach Anspruch 7, 9 oder 10, wobei die Hülle mit einer weiteren Hülle (20) versehen ist, in der die Stromversorgung untergebracht werden kann.

12. Kombination nach einem oder mehreren der vorstehenden Ansprüche, wobei die Heizmittel mit Zeiteinstellmitteln zum Einstellen einer Heizzeit versehen sind.

13. Kombination nach einem oder mehreren der vorstehenden Ansprüche, wobei die Heizmittel mit Temperatureinstellmitteln zum Einstellen einer Heiztemperatur versehen sind.

14. Kombination nach einem oder mehreren der vorstehenden Ansprüche, wobei die Hülle aus einem Gewebe hergestellt ist.

## Revendications

1. Combinaison d'un support (10) pour un pansement de stomie et d'un pansement de stomie, ledit support comprenant une enveloppe composée de deux couches (11, 12), lesquelles couches sont assemblées au niveau de leurs bords circonférentiels et ne sont pas assemblées au niveau d'un bord circonférentiel, laquelle enveloppe entoure un espace (15), dans cet espace (15) le pansement de stomie est placé, **caractérisée en ce que** le support comprend également des moyens de chauffage pour chauffer le pansement de stomie, où les moyens de chauffage (25) comprennent un ou plusieurs fil(s) chauffant(s) (25-25'-250), qui sont agencés dans l'enveloppe sur toute la surface de couche interne de l'enveloppe sous la forme de plusieurs enveloppes ou enroulements, permettant d'obtenir ainsi une surface de contact maximale entre la couche interne de l'enveloppe et le pansement de stomie qui est placé dans l'espace (15).

2. Combinaison selon la revendication 1, dans laquelle l'enveloppe peut être fermée.

3. Combinaison selon la revendication 2, dans laquelle l'enveloppe pouvant être fermée est munie d'une fermeture à glissière (22).

4. Combinaison selon la revendication 2, dans laquelle l'enveloppe pouvant être fermée est munie d'un ou de plusieurs bouton(s) à pression.

5. Combinaison selon la revendication 2, dans laquelle l'enveloppe pouvant être fermée est munie d'une fermeture magnétique.

6. Combinaison selon la revendication 2, dans laquelle l'enveloppe pouvant être fermée est munie d'une fermeture autoagrippante.

7. Combinaison selon une ou plusieurs des revendications précédentes, dans laquelle les moyens de chauffage comprennent en outre une alimentation électrique qui est reliée aux fils chauffants.

8. Combinaison selon la revendication 7, dans laquelle l'alimentation électrique peut être activée et désactivée.

9. Combinaison selon la revendication 7 ou 8, dans laquelle l'alimentation électrique est une connexion 110V/220V.

10. Combinaison selon la revendication 7 ou 8, dans laquelle l'alimentation électrique est rechargeable.

11. Combinaison selon la revendication 7, 9 ou 10, dans laquelle l'enveloppe est munie d'une enveloppe supplémentaire (20), dans laquelle l'alimentation électrique peut être reçue.

12. Combinaison selon une ou plusieurs des revendications précédentes, dans laquelle les moyens de chauffage sont munis de moyens de réglage de temps pour régler un temps de chauffage.

13. Combinaison selon une ou plusieurs des revendications précédentes, dans laquelle les moyens de chauffage sont munis de moyens de réglage de température pour régler une température de chauffage.

14. Combinaison selon une ou plusieurs des revendications précédentes, dans laquelle l'enveloppe est réalisée en un tissu.
